## Europäisches Patentamt
## European Patent Office
## Office européen des brevets

(19)

(11) Veröffentlichungsnummer: **0 157 022**
**B1**

(12) # EUROPÄISCHE PATENTSCHRIFT

(45) Veröffentlichungstag der Patentschrift:
27.04.88

(51) Int. Cl.⁴: **C 07 D 311/92**

(21) Anmeldenummer: **84201856.6**

(22) Anmeldetag: **13.12.84**

(54) **Verfahren zur Herstellung von 4-Chlor-bzw.4-Bromnaphthal säureanhydrid.**

(30) Priorität: **27.03.84 DE 3411196**

(43) Veröffentlichungstag der Anmeldung:
**09.10.85 Patentblatt 85/41**

(45) Bekanntmachung des Hinweises auf die Patenterteilung:
**27.04.88 Patentblatt 88/17**

(84) Benannte Vertragsstaaten:
**BE CH DE FR GB IT LI**

(56) Entgegenhaltungen:
**DE-A-2 242 513**
**DE-A-2 302 372**
**US-A-4 033 986**

**Die Akte enthält technische Angaben, die nach
dem Eingang der Anmeldung eingereicht wurden
und die nicht in dieser Patentschrift enthalten sind.**

(73) Patentinhaber: **RÜTGERSWERKE
AKTIENGESELLSCHAFT, Mainzer Landstrasse 217,
D-6000 Frankfurt am Main 1 (DE)**

(72) Erfinder: **Orth, Winfried, Dr., Am Schachtelgraben
28, D-6733 Hassloch/Pfalz (DE)**
Erfinder: **Pastorek, Emmerich, Dr.,
Grünbergerstrasse 90, D-6944 Hemsbach (DE)**
Erfinder: **Fickert, Werner, Dr., Stockacher Strasse
14, D-6800 Mannheim 61 (DE)**

## Beschreibung

Die Erfindung betrifft ein neues, vereinfachtes Verfahren zur Herstellung von in 4-Stellung mit Chlor bzw. Brom substituierten Naphthalsäureanhydriden. Diese Verbindungen sind wertvolle Zwischenprodukte bei der Herstellung von Farbstoffen und optischen Aufhellern.

Der einfachste und in Bezug auf die Wirtschaftlichkeit, auch hinsichtlich der Ausgangsstoffe, günstigste Syntheseweg besteht in der direkten Chlorierung bzw. Bromierung.

Die relativ große Anzahl an entsprechenden vorbeschriebenen Verfahren zeigt, daß dabei die Wahl der Reaktionsbedingungen und insbesondere die Wahl des pH-Wertes der Reaktionslösung von entscheidendem Einfluß auf den Reaktionsverlauf und damit auf Ausbeute und Reinheit der Endprodukte sind.

So beschreiben Rule and Thomson (J. Chem. Soc. 1937, 1764 - 67), daß die Bromierung von Naphthalsäureanhydrid in saurem Medium zu 3-Bromnaphthalsäureanhydrid und in alkalischem Medium zu 4-Bromnaphthalsäureanhydrid führt. Aber auch in alkalischem Medium, in dem das Alkalisalz der Naphthalsäure vorliegt, wird nur eine Ausbeute von 43 % 4-Bromnaphthalsäureanhydrid in mäßiger Reinheit erzielt. Eine Verschärfung der Reaktionsbedingungen führt zur Bildung von Tribromnaphthalsäureanhydrid.

Nach DE-PS-2 242 513 und DE-OS-2 302 372 sind die optimalen Bedingungen sowohl für die Chlorierung wie auch die Bromierung von Naphthalsäureanhydrid (bzw. Alkalisalz der Naphthalsäure) eine Reaktionstemperatur im Bereich von 0 bis 30° C und ein pH-Wert des Reaktionsmediums im Bereich von 6,8 - 9,0, bevorzugt im Bereich von 7,2 - 7,8.

Nach US-PS-4 033 986 wird das Dialkalisalz der Naphthalsäure bei einem pH-Wert im Bereich von 6,5 bis 9,5 und einer Temperatur im Bereich von 0 bis 30° C bromiert.

Auch gemäß der japanischen Anmeldung 77 151 152 (C.A. 88, 120 868c) wird 4-Chlornaphthalsäureanhydrid durch Chlorierung bei einem pH-Wert im Bereich 6,8 - 9,0, allerdings bei anderen Temperaturen, hergestellt. Da während der Reaktion eine pH-Verschiebung eintritt, ist bei allen diesen Verfahren das Einhalten eines derart engen pH-Bereiches durch fortlaufende gezielte Neutralisation während der Reaktion notwendig und sehr aufwendig.

Dieser Nachteil wird beim Verfahren nach der japanischen Anmeldung 75 58 042 (C.A. 83, 114076g) überwunden. Danach erfolgt die Chlorierung von Alkalinaphthalat in Alkalihydroxidlösungen. Dabei wird allerdings eine um 20 Prozentpunkte verminderte Ausbeute an 4-Chlornaphthalsäureanhydrid erhalten und der Anteil an gebildetem Dichlornaphthalsäureanhydrid ist relativ hoch. Außerdem zeigten einige Versuche, daß bei dieser Umsetzung verhältnismäßig viel (4 %) unerwünschtes 3-Chlornaphthalsäureanhydrid entsteht.

Es ist daher Aufgabe der Erfindung, ein Verfahren zur Herstellung von 4-Chlor- bzw. 4-Bromnaphthalsäureanhydrid bereitszustellen, nach dem diese Produkte durch einfache Chlorierung bzw. Bromierung ohne aufwendige Einhaltung enger pH-Bereiche in möglichst hoher Ausbeute und hoher Reinheit gewonnen werden.

Die Lösung erfolgt durch ein Verfahren gemäß der Ansprüche 1 bis 3.

Obwohl bei diesen Verfahren der pH-Wert des Reaktionsgemisches während der Chlorierung bzw. Bromierung je nach Wahl der Alkalimengen im Bereich von 9,5 bis 13 liegt, wird überraschenderweise 4-Chlor- bzw. 4-Bromnaphthalsäureanhydrid in hoher Ausbeute und Reinheit gewonnen. Die Chlorierung bzw. Bromierung erfolgt einfach durch Einleiten von molekularem Chlor oder Brom oder durch Zugabe von Hypochlorit bzw. Hypobromit ohne weitere Kontrolle oder Regulierung des pH-Wertes der Reaktionslösung. Ein wirtschaftliches Bromierungsmittel ist die als Bromchlorid bekannte Verbindung der beiden Halogene Chlor und Brom miteinander.

Eine zusätzliche Erhöhung der Reaktionsgeschwindigkeit, der Ausbeute und der Reinheit wird erzielt, wenn die Chlorierung bzw. Bromierung unter intensivem Vermischen in Gegenwart eines wasserunlöslichen, gegen Halogen inerten Lösemittels durchgeführt wird.

Es ist einleuchtend, daß mit Hilfe eines derartigen Lösemittels die Verteilung des Chlors bzw. Broms und damit die Chlorierungs- bzw. Bromierungsreaktion erleichtert werden. Um so überraschender ist es, daß durch diese Maßnahme die Bildung unerwünschter Dichlor- oder Dibromderivate nicht erhöht sondern geringfügig vermindert wird. Ein weiterer unvorhergesehener Vorteil bei der Verwendung derartiger Lösemittel besteht darin, daß die an sich schwer filtrierbaren 4-Chlor- bzw. 4-Bromnapthalsäureanhydridverbindungen bei dieser erfindungsgemäßen Verfahrensvariante in einer leicht filtrierbaren Form anfallen.

Ausgangsprodukt für das Verfahren ist Naphthalsäureanhydrid, das als Reinsubstanz oder aber auch als technische Qualität eingesetzt werden kann. Das Naphthalsäureanhydrid wird zusammen mit einer äquivalenten Menge Alkalihydroxid, bevorzugt Kaliumhydroxid, in Wasser, vorzugsweise in der Wärme gemischt und geht als Alkalisalz der Naphthalsäure (Alkalinaphthalat) in Lösung.

Diese Lösung wird auf 10 bis 25° C abgekühlt und mit einem wasserlöslichen Magnesiumsalz und Alkalihydroxid versetzt. Dabei fällt Magnesiumhydroxid in feinst verteilter Form aus. Als Magnesiumsalze eignen sich alle wasserlöslichen Salze oder Salzgemische. Aus wirtschaftlichen Gründen wird vorzugsweise Magnesiumchlorid oder -sulfat verwendet. Obwohl bei der Umsetzung mit Alkalihydroxid

2

unlösliches Magnesiumhydroxid entsteht, ist es nicht ratsam, direkt festes pulverförmiges Magnesiumhydroxid einzusetzen. Eingesetzt werden sollte mindestens die dem Naphthalsäureanhydrid entsprechende molare Menge, wobei ein Überschuß von 10 bis 50 % bevorzugt wird. Aber auch ein höherer Überschuß stört nicht, wenn man von der mangelnden Wirtschaftlichkeit absieht. Das Magnesiumsalz kann als festes Salz oder bereits gelöst in Wasser eingesetzt werden. Die Menge der einzusetzenden Alkalilauge soll der des Magnesiumsalzes mindestens äquivalent sein. Bevorzugt wird auch hier ein Überschuß von 10 bis 20 % eingesetzt. Je nach Alkalihydroxidmenge erhält man einen pH-Wert der Reaktionsmischung von 11 bis 13.

In die so hergestellte Reaktionslösung wird bei einer Temperatur im Bereich von 10 bis 25°C Chlor bzw. Brom oder Hypochlorit- bzw. Hypobromitlösung eingeleitet oder über die Lösung Chlor bzw. Brom geleitet, wobei die Halogenmenge bevorzugt weniger als die doppelte molare Menge des eingesetzten Naphthalsäureanhydrids betragen soll. Nach der Chlor- bzw. Bromzugabe wird die Reaktionsmischung noch etwa 1 bis 3 h gerührt, wobei gegebenenfalls die Reaktionstemperatur bis auf etwa 50°C erhöht werden kann. Danach wird mit einer starken Säure angesäuert und das ausgefallene Produkt in an sich bekannter Weise abgetrennt und gereinigt.

Eine weitere erfindungsgemäße Verbesserung des Verfahrens besteht darin, die Reaktionslösung oder -mischung mit etwa 5 bis 15 Vol.-% eines in Wasser unlöslichen und gegen Halogen unter den gegebenen Reaktionsbedingungen gegen Halogen inerten Lösemittel zu versetzen und unter intensivem Mischen die Halogenierungsreaktion durchzuführen. Bevorzugte derartige Lösemittel sind niedere Halogenalkane oder Halogenaryle wie z.B. Methylenchlorid, Tetrachlormethan, Äthylenchlorid, Trichloräthan, Trichlorpropan oder Chlorbenzol. Diese Lösemittel werden nach beendeter Umsetzung durch Destillation zurückgewonnen und können in einem weiteren Reaktionsansatz wiederverwendet werden.

**Beispiel 1**

297 g (1,5 Mol) Naphthalsäureanhydrid werden in 4500 ml Wasser und 240 g (3 Mol) 50-%-iger Natronlauge unter Rühren bei 80°C gelöst. Zu der auf 20 - 25°C abgekühlten Lösung gibt man auf einmal eine Lösung, hergestellt aus 813 g (3,3 Mol) Magnesiumsulfat 7 H₂O und 1200 ml Wasser. Gleich danach werden 288 g (3,6 Mol) 50-%-iger Natronlauge langesam zugetropft und es fällt Magnesiumhydroxid aus. Zu dieser Suspension werden 200 ml Äthylenchlorid zugegeben und innerhalb von 4 1/2 Stunden bei 20 - 25°C 198 g (2,8 Mol) Chlor über die Oberfläche des Reaktionsgemisches geleitet. Danach rührt man noch 1 Stunde nach. Das Reaktionsgemisch wird nach der Nachreaktion vorsichtig mit ca. 370 ml (4,4 Mol) konzentrierter Salzsäure auf pH 1 bis 0,5 gebracht und das Äthylenchlorid abdestilliert. Das ausgefallene Produkt wird bei ca. 40°C abgenutscht, sulfatfrei gewaschen und bei 60-110°C getrocknet.
Ausbeute: 302 g = 86,8 % der Theorie,
Reinheit: (gaschromatographisch) 94 - 95 %.

**Beispiel 2**

4-Chlornaphthalsäureanhydrid wird analog Beispiel 1 hergestellt, jedoch ohne Verwendung von Äthylenchlorid. Nach Aufarbeitung des Reaktionsgemisches werden folgende Ergebnisse erzielt:
Ausbeute: 288 g = 82,8 % der Theorie
Gehalt (gaschromatographisch): 91 %

**Patentansprüche**

1. Verfahren zur Herstellung von 4-Chlor- bzw. 4-Bromnaphthalsäureanhydrid durch Chlorierung bzw. Bromierung einer wäßrigen Lösung eines Alkalisalzes der Naphthalsäure mit Chlor bzw. Brom oder Hypochlorit bzw. Hypobromit, dadurch gekennzeichnet, daß eine Lösung des Alkalisalzes der Naphthalsäure mit mindestens der äquimolaren Menge eines wasserlöslichen Magnesiumsalzes und der mindestens äquimolaren Menge eines Alkalihydroxids versetzt und dieses Reaktionsgemisch bei einem pH-Wert im Bereich von 11-13 und einer Temperatur im Bereich von 10 bis 25°C chloriert bzw. bromiert und anschließend angesäuert wird.

2. Verfahren nach Anspruch 1, dadurch gekennzeichnet, daß die Chlorierung bzw. Bromierung unter intensivem Vermischen in Gegenwart eines niedrigen Halogenalkans oder Halogenaryls bevorzugt von Methylenchlorid, Trichlormethan, Tetrachlormethan, Äthylenchlorid, Trichloräthan, Trichlorpropan oder Chlorbenzol durchgeführt wird.

3. Verfahren zur Herstellung von 4-Bromnaphthalsäureanhydrid nach einem der Ansprüche 1 bis 2, dadurch gekennzeichnet, daß als Bromierungsmittel Bromchlorid verwendet wird.

**Claims**

1. A process for the preparation of 4-chloro- or 4-bromonaphthalic acid anhydride by chlorination or bromination respectively of an aqueous solution of an alkali salt of naphthalic acid with chlorine or bromine respectively or hypochlorite or hypobromite respectively, characterized in that a solution of the alkali salt of the naphthalic acid is reacted with at least the equimolar quantity of a water-soluble magnesium salt and the at least equimolar quantity of an alkali hydroxide, and this reaction mixture is chlorinated or brominated respectively at a pH value of in the range of from 11 to 13 and at a temperature in the region from 10 to 25°C and is then acidified.

2. A process according to Claim 1, characterized in that the chlorination or bromination respectively is performed with intensive mixing in the presence of a low halogen alkane or halogen aryl preferably of methylene chloride, trichloromethane, tetrachloromethane, ethylene chloride, trichloroethane, trichloropropane or chlorobenzene.

3. A process for the preparation of 4-bromonaphthalic acid anhydride according to one of Claims 1 to 2, characterized in that bromine chloride is used as a brominating agent.

**Revendications**

1. Procédé de préparation d'anhydride de l'acide 4-chloro ou 4-bromonaphtalique par chloruration ou bromuration d'une solution aqueuse d'un sel alcalin de l'acide naphtalique à l'aide de chlore ou de brome ou de l'hypochlorite ou de l'hypobromite, caractérisé par le fait qu'une solution du sel alcalin de l'acide naphtalique est mise à réagir avec au moins la quantité équimolaire d'un sel de magnésium soluble dans l'eau et avec une quantité au moins équimoléculaire d'un hydroxyde alcalin, ce mélange réactionnel étant chloruré ou bromuré à une valeur de pH de 11 à 13 et à une température située dans le domaine de 10 à 25°C, et subséquemment acidifié.

2. Procédé selon la revendication 1, caractérisé en ce que la chloruration ou bromuration est effectuée sous mélange intensif en présence d'un alcane halogéné inférieur ou d'un aryle halogéné, de préférence en présence de chlorure de méthylène, de trichlorométhane, de tétrachlorométhane, de chlorure d'éthylène, de trichloréthane, de trichloropropane ou de chlorobenzène.

3. Procédé de préparation d'anhydride de l'acide 4-bromonaphtalique selon l'une des revendications 1 à 2, caractérisé par le fait qu'on utilise du chlorure de brome en tant qu'agent de bromuration.